(19) Europäisches Patentamt
European Patent Office
Office européen des brevets

(11) **EP 4 537 759 A1**

(12) **EUROPEAN PATENT APPLICATION**

(43) Date of publication:
16.04.2025 Bulletin 2025/16

(21) Application number: 24206505.0

(22) Date of filing: 14.10.2024

(51) International Patent Classification (IPC):
A61B 6/03 (2006.01)  A61B 6/50 (2024.01)
A61B 6/00 (2024.01)  G06T 11/00 (2006.01)
G06T 5/70 (2024.01)

(52) Cooperative Patent Classification (CPC):
A61B 6/032; A61B 6/503; A61B 6/5264;
A61B 6/5288; A61B 6/541; A61B 6/545;
G06T 5/70; G06T 11/005; G06T 11/008;
G06T 2207/10081; G06T 2211/412

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 12.10.2023 JP 2023176865

(71) Applicant: FUJI-FILM Corporation
Minato-ku
Tokyo 106-8620 (JP)

(72) Inventors:
• MURASE, Takashi
  Tokyo, 106-8620 (JP)
• TETSUMURA, Yusuke
  Tokyo, 106-8620 (JP)

(74) Representative: Dehns Germany Partnerschaft
mbB
Theresienstraße 6-8
80333 München (DE)

(54) **X-RAY CT APPARATUS, IMAGE PROCESSING DEVICE, AND MOTION-CORRECTED IMAGE RECONSTRUCTION METHOD**

(57)    In a case where motion information of a subject (3) during scanning is acquired from an image pair, and a CT image is generated by performing motion-corrected image reconstruction, erroneous recognition of a motion component and excessive smoothing are suppressed to improve accuracy of motion correction.

A motion information acquisition unit (330) includes a filtering unit (331) that reduces noise of an image pair, a registration unit (333) that performs registration processing by using the image pair after filtering, and a processing condition adjustment unit (335) that adjusts conditions of filtering processing and the registration processing in accordance with a tube current ratio of the image pair. The processing condition adjustment unit refers to a table in which a relationship between the tube current ratio and a parameter of each processing is predetermined, selects parameters corresponding to a tube current ratio of a target image pair, and determines the parameters for filtering and registration. The motion information acquisition unit acquires motion information by performing filtering and registration by using the determined parameters, and the image reconstruction unit performs motion-corrected image reconstruction by using the motion information.

FIG. 2

EP 4 537 759 A1

## Description

The present application claims priority to Japanese Patent Application No. 2023-176865, filed October 12, 2023.

## BACKGROUND OF THE INVENTION

### 1. Field of the Invention

The present invention relates to an X-ray CT apparatus that obtains a medical image by irradiating a subject with X-rays, and particularly, to a motion-corrected image reconstruction processing technique for improving image interpretation accuracy and image interpretation efficiency for a moving subject.

### 2. Description of the Related Art

In a CT examination for a moving subject such as a heart, motion artifacts may occur in an image due to movements of the subject during a CT scan. These motion artifacts degrade image quality, which may result in reduced diagnostic accuracy and reduced diagnostic efficiency of a disease by a doctor, an examination technician, and the like (hereinafter, collectively referred to as an examiner). Therefore, motion-corrected image reconstruction processing is performed to reduce the motion artifacts occurring in a CT image of the moving subject.

In the motion-corrected image reconstruction processing, reconstruction is performed by estimating the motion of the subject from a pair of images (a first image and a second image) reconstructed at temporally directly opposite positions with a target image reconstruction position as a center and by performing back projection while correcting an image at a target reconstruction position by using information on the estimated motion. By applying such motion-corrected image reconstruction, it is possible to correct heart motion that cannot be resolved by simply matching cardiac phase conditions, organ motion in ECG-asynchronous imaging, and the like.

However, in a case where there is noise in two images used to estimate the motion, there are problems such as erroneously recognizing noise as motion or failing to detect motion that is obscured by noise. US10165989B2 discloses a method of extracting only motion of a subject by performing noise reduction processing on a first image and a second image. In the method disclosed in US10165989B2, noise reduction is performed by applying a low-pass filter, based on an X-ray dose in a case where the first image and the second image are acquired.

## SUMMARY OF THE INVENTION

In the method disclosed in US 10165989B2, noise is reduced in accordance with the noise in each of the first image and the second image, but a relation of noise between the images is not considered. Noise increases in a case where a tube current is low at the time of the acquisition of transmitted X-ray data. However, in a case where there is a significant difference in the tube current at the time of acquisition of an image pair, excessive smoothing may be performed due to the influence of a noise component in a low-dose region, resulting in problems such as distortion in a streak component. On the other hand, in a case where over-correction such as excessive smoothing is suppressed too much, phases that could originally have remained stationary are affected.

Therefore, an object of the present invention is to suppress erroneous recognition of a motion component and excessive smoothing mentioned above, and to improve accuracy of motion correction.

In order to achieve the above-described object, the present invention relates to adjusting conditions of processing performed at the time of acquisition of motion information, for a pair of images used to obtain the motion information, that is, a first image and a second image (hereinafter, also referred to as an image pair), in accordance with a ratio between tube currents in a case where each image of the image pair has been acquired. The main processing performed at the time of the acquisition of the motion information is noise reduction processing of the image and registration processing after noise reduction, and the adjustment of the conditions of the processing can be performed by adjusting types of algorithms used in the processing or parameters included therein.

That is, an X-ray CT apparatus of an aspect of the present invention includes: an imaging unit that includes an X-ray source and an X-ray detector which rotate around a subject and that acquires transmitted X-ray data of the subject in a predetermined angular range; and an image reconstruction unit that generates a reconstructed image by using the transmitted X-ray data acquired by the imaging unit. The image reconstruction unit includes an image pair generation unit that generates a first image and a second image at directly opposite positions by using a part of the transmitted X-ray data, and a motion information acquisition unit that performs noise reduction processing on the first image and the second image and registration processing between the first image and the second image after noise reduction and that calculates motion information of the subject during scanning, and the image reconstruction unit generates the reconstructed image by correcting motion of the subject during scanning using the motion information calculated by the motion information acquisition unit.

According to a first aspect of the present invention, the motion information acquisition unit includes a processing condition adjustment unit that classifies an image pair consisting of the first image and the second image, in accordance with a ratio between tube currents in a case where the transmitted X-ray data used to generate the first image and the second image has been acquired, and that adjusts conditions of the noise reduction processing and the registration processing for each classification.

In addition, according to a second aspect of the present invention, the motion information acquisition unit includes a processing condition adjustment unit that adjusts a parameter of a non-rigid registration algorithm, in accordance with a ratio between tube currents in a case where the transmitted X-ray data used to generate the first image and the second image has been acquired.

An image processing device of another aspect of the present invention is a device having a function of the image reconstruction unit of the X-ray CT apparatus.

Further, a motion-corrected image reconstruction method of still another aspect of the present invention is a method of performing image reconstruction of a CT image by correcting motion of a subject during scanning by using transmitted X-ray data, the method including: a step of generating a first image and a second image at directly opposite positions by using a part of the transmitted X-ray data; a step of acquiring motion information of the subject by performing noise reduction processing and registration processing after noise reduction, on each of the first image and the second image; and a step of performing image reconstruction by using the motion information and the transmitted X-ray data. In the step of acquiring the motion information, at least one of a type or a parameter of a noise reduction filter used in the noise reduction processing and a parameter of a non-rigid registration algorithm used in the registration processing are adjusted based on a ratio between tube currents in a case where the first image and the second image have been acquired.

According to the aspects of the present invention, in a case of performing motion-corrected image reconstruction by using the motion information acquired from the image pair, excessive correction caused by a difference in the tube current acquired for each image of the image pair can be prevented, and an image in which the motion of the subject is properly corrected and the influence on a stationary portion is minimized can be obtained.

**BRIEF DESCRIPTION OF THE DRAWINGS**

Fig. 1 is a diagram showing an overall configuration of an X-ray CT apparatus of an embodiment of the present invention.

Fig. 2 is a diagram showing components of the X-y CT apparatus of the embodiment of the present invention.

Fig. 3 is a functional block diagram of an image reconstruction unit of the embodiment.

Fig. 4 is a diagram showing a flow of processing of the X-ray CT apparatus of Embodiment 1.

Fig. 5 is a diagram showing a relationship between a first image and a second image with respect to a targeted reconstruction position.

Fig. 6A is a diagram showing a relationship between electrocardiogram waveform data and magnitude of a tube current value in electrocardiogram-synchronized imaging and is a diagram showing an electrocardiogram waveform and a targeted imaging cardiac phase.

Fig. 6B is a diagram showing the relationship between the electrocardiogram waveform data and the magnitude of the tube current value in the electrocardiogram-synchronized imaging and is a diagram showing a relationship between an imaging cardiac phase and a tube current.

Fig. 6C is a diagram showing the relationship between the electrocardiogram waveform data and the magnitude of the tube current value in the electrocardiogram-synchronized imaging and is a diagram showing the relationship between the imaging cardiac phase and the tube current.

Fig. 7 is a diagram illustrating a tube current ratio.

Fig. 8 is a diagram showing an example of a table that defines a relationship between the tube current ratio and a parameter set of Embodiment 1.

Fig. 9 is a diagram showing an example of filter characteristics and parameters of non-rigid registration after adjustment.

Fig. 10 is a diagram showing details of parameter adjustment of Embodiment 1.

Fig. 11 is a diagram showing processing of Embodiment 2.

Fig. 12 is a diagram illustrating determination of presence or absence of motion.

**DESCRIPTION OF THE PREFERRED EMBODIMENTS**

Hereinafter, embodiments of the present invention will be described with reference to the drawings.

First, an overall configuration of an X-ray CT apparatus to which the present invention is applied will be described.

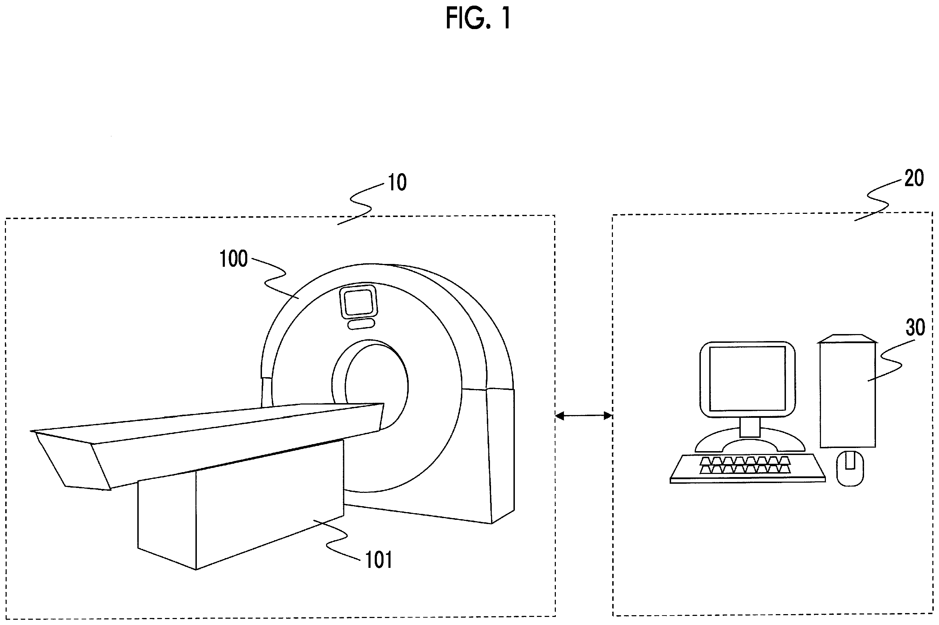

As shown in Fig. 1, an X-ray CT apparatus 1 includes an imaging unit 10 that includes a gantry 100 for capturing a tomographic image and a fluoroscopic image of a subject 3 and a patient table device 101, and an operation unit 20 for operating and controlling the imaging unit 10.

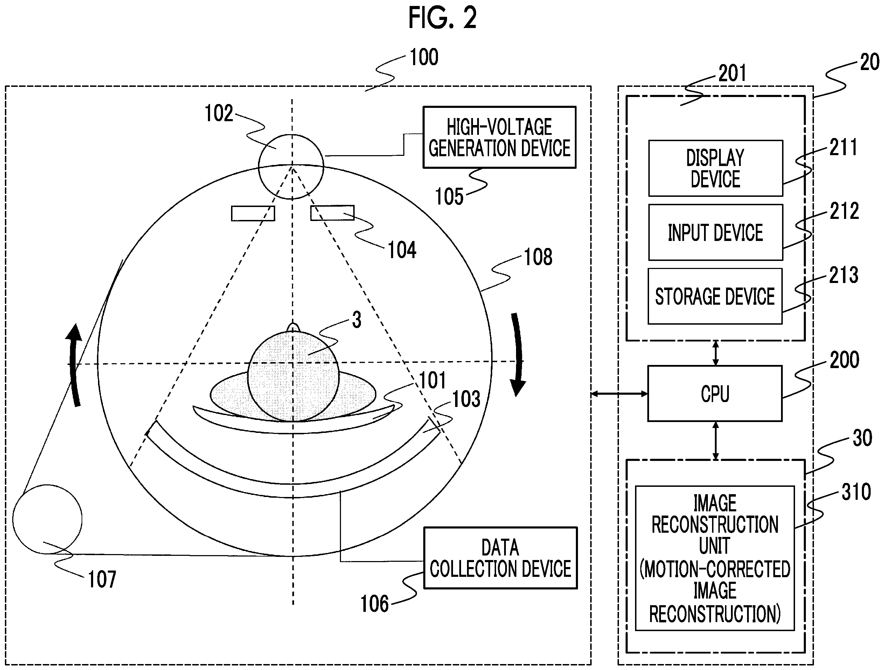

As shown in Fig. 2, the gantry 100 includes an X-ray generation device 102 that is equipped with an X-ray tube that generates X-rays for irradiating the subject 3, a collimator device 104 that narrows a beam of X-rays generated from the X-ray generation device 102, an X-ray detection device 103 that detects X-rays transmitted through the subject, a scanner 108 that is equipped with the X-ray generation device 102, the collimator device 104, and the X-ray detection device 103, a high-voltage generation device 105 that applies a high voltage to the X-ray generation device 102, a data collection device 106 that collects transmitted X-ray data obtained from the X-ray detection device 103, and a drive device 107 that rotates the scanner around the subject 3. Although not shown, the X-ray generation device 102 includes the X-ray tube, and a predetermined tube current flows through the X-ray tube, thereby irradiating the subject 3 with a predetermined dose of X-rays. In a case of ECG-synchronized imaging, imaging is performed based on information from an electrocardiograph (not shown) attached to the subject 3, and in a case of asynchronous imaging, imaging is performed under automatic exposure control.

The operation unit 20 includes a central control unit (CPU) 200 that controls each device built into the gantry and an input/output device 210 that functions as a user interface for interaction between the user and the CPU 200, and the CPU 200 is equipped with a calculation unit 30 that performs various calculations, such as image reconstruction, on the transmitted X-ray data collected by the data collection device 106. However, a separate calculation device from the CPU 200 may be provided, and this calculation device may function as the calculation unit 30. The functions of the CPU 200 are implemented by the CPU 200 reading and executing programs that describe calculation algorithms and control processing procedures, but some of the calculations and processing performed by the calculation unit 30 can also be performed by using a programmable logic device (PLD) such as an ASIC or an FPGA.

The input/output device 210 includes an input device 212 that is used for an operator to input imaging conditions and the like, a display device 211 that displays data, such as captured images, and a GUI, and a storage device 213 that stores data required for imaging, such as programs and device parameters.

The CPU 200 controls the imaging unit 10 (the X-ray generation device 102, the X-ray detection device 103, the high-voltage generation device 105, the collimator device 104, the patient table device 101, the drive device 107, and the data collection device 106), the input/output device 210, and the calculation unit 30 in response to operation instructions from the operator via the input device 212. Under the control of the CPU 200, the respective units operate to perform reconstruction of a CT image, correction of the reconstructed CT image, and the like.

In a case of the ECG-synchronized imaging, the information from the electrocardiograph (not shown) attached to the subject 3 is sent to the CPU 200, and the CPU 200 controls the high-voltage generation device 105 in synchronization with the phase of the heart motion to control the tube current to be supplied to the X-ray generation device (X-ray tube). In addition, in a case of asynchronous imaging, an automatic exposure control function is provided, and the tube current is automatically controlled. Through such control of the tube current, the dose is adjusted to prevent continuous high doses, thereby reducing unnecessary radiation exposure.

The calculation unit 30 includes an image reconstruction unit 310 that performs back projection processing on the transmitted X-ray data obtained by the data collection device 106 to create a tomographic image. The image reconstruction unit 310 includes not only normal image reconstruction but also motion-corrected image reconstruction in imaging targeting moving parts. The imaging targeting moving parts includes not only imaging synchronized with a phase (cardiac phase) of the heart motion, such as ECG-synchronized imaging, but also ECG-asynchronous imaging that includes a moving organ.

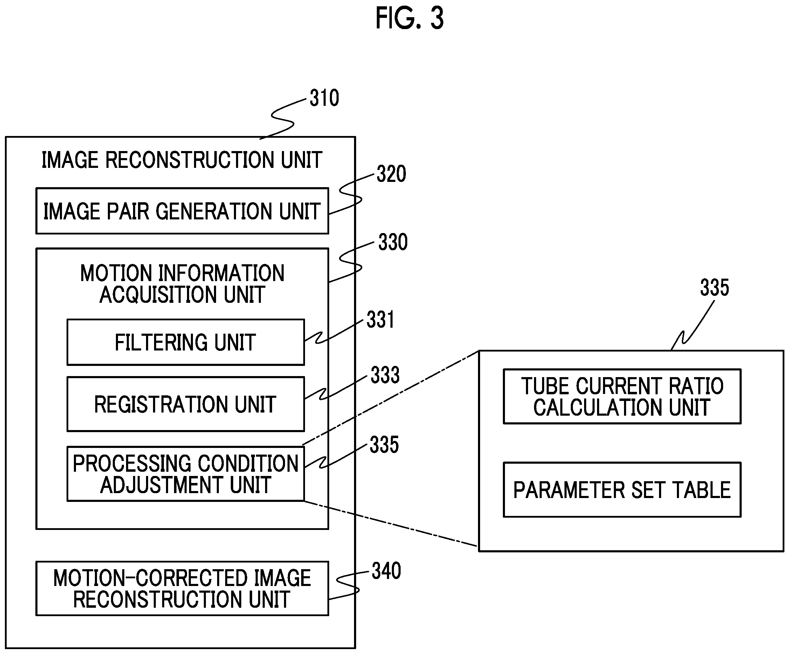

As shown in Fig. 3, in order to perform motion correction reconstruction, the image reconstruction unit 310 includes an image pair generation unit 320 that generates a first image and a second image (hereinafter, a pair consisting of the first image and the second image is also referred to as an image pair) at directly opposite positions by using a part of the transmitted X-ray data, a motion information acquisition unit 330 that extracts the motion of the subject by using the image pair, and a motion-corrected image reconstruction unit 340.

The motion information acquisition unit 330 acquires motion information by performing processing for reducing noise included in the image pair (noise reduction processing) and registration processing on the image pair after noise reduction. In this case, the motion information acquisition unit 330 acquires information on the tube current in a case where the transmitted X-ray data of the image pair has been acquired from the imaging unit 10, calculates a ratio between the tube currents of two pieces of transmitted X-ray data, and adjusts the conditions of the noise reduction processing and the registration processing by using the calculated ratio. Although the adjustment method will be described in detail in the embodiment to be described below, there is a method of classifying the image pair in accordance with the tube current ratio and selecting a condition set in advance in accordance with the classification, or a method of adjusting a type or a parameter of the algorithm used in each processing in accordance with the tube current ratio.

Hereinafter, an embodiment of processing of the X-ray CT apparatus in a case of performing the motion-corrected image reconstruction will be described.

Embodiment 1

As shown in Fig. 3, the motion information acquisition unit 330 of the present embodiment includes a filtering unit 331 that performs noise reduction processing, a registration unit 333 that performs registration processing, and a processing condition adjustment unit 335 that adjusts the condition of each processing in accordance with the tube current ratio of the image pair. The processing condition adjustment unit 335 includes a tube current ratio calculation unit that calculates the tube current ratio of the image pair, and a table in which the relationship between the tube current ratio and the conditions of filtering and registration processing is set in advance.

In the present embodiment, as an example, a case where the conditions of the processing, particularly the parameters, are adjusted for both the noise reduction processing and the registration processing will be described.

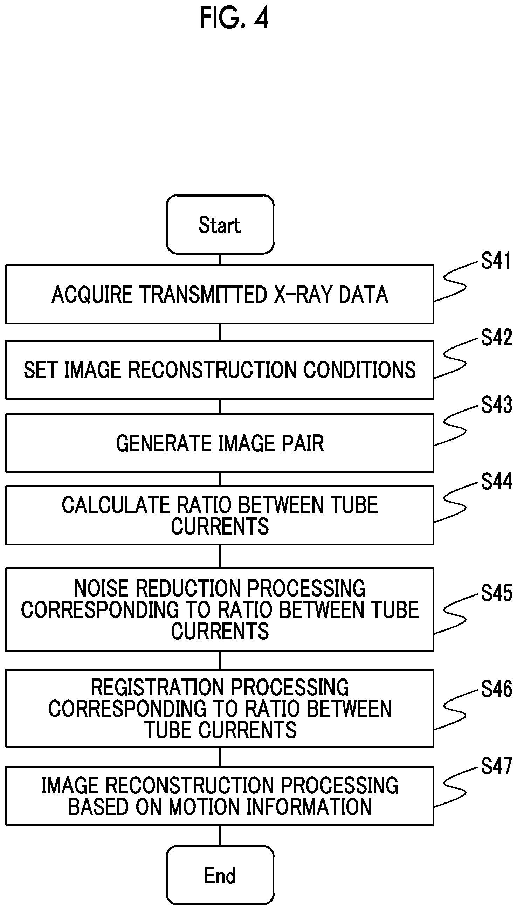

Hereinafter, a flow of processing of the present embodiment will be described with reference to Fig. 4.

Imaging: S41

Positioning imaging is performed while the subject 3 is placed on the patient table device 101. The positioning imaging is imaging for setting an imaging range of the subject 3 and allows a transmitted X-ray image to be acquired along a body axis direction while changing a relative position between the scanner 108 and the patient table device 101 (subject 3). An examiner sets the imaging range by using the transmitted X-ray image. Next, the imaging unit 10 performs tomographic imaging with the rotation of the scanner 108 within the imaging range set based on a positioning image and collects the transmitted X-ray data of the subject.

Image Reconstruction Condition Setting: S42

Image reconstruction conditions for the transmitted X-ray data of the subject, which is acquired in the imaging step S41, are set. The image reconstruction conditions include, for example, an image thickness (cross-section thickness), FOV, filter conditions, and the like, and in a case of the ECG-synchronized imaging, further include settings such as a reconstruction cardiac phase (target reconstruction cardiac phase: which cardiac phase the image is to be reconstructed at). In the ECG-synchronized imaging, the target reconstruction cardiac phase is set, thereby determining a target reconstructed image position. In a case of the ECG-asynchronous imaging, the user determines the target reconstructed image position. The image reconstruction unit 310 accepts these image reconstruction conditions set by the user via the input device 212.

Motion-Corrected Image Reconstruction S43 to S47

The image reconstruction unit 310 performs image reconstruction by using the transmitted X-ray data of the subject, which is acquired in the imaging step S41, based on the image reconstruction conditions set in the image reconstruction condition setting step S42. In this case, motion information of the subject during imaging is acquired, and the motion is corrected, thereby performing reconstruction (motion-corrected image reconstruction)

Image Pair Generation: S43

In the motion-corrected image reconstruction, first, the image pair generation unit 320 uses a filter correction back projection method to generate two images, that is, the first image and the second image, from the transmitted X-ray data collected by the data collection device 106 of the imaging unit 10. The two images constitute a directly opposite image pair with the target reconstructed image position as the center.

The first image and the second image are each not limited to being one two-dimensional image and may be a three-dimensional image composed of a plurality of two-dimensional images.

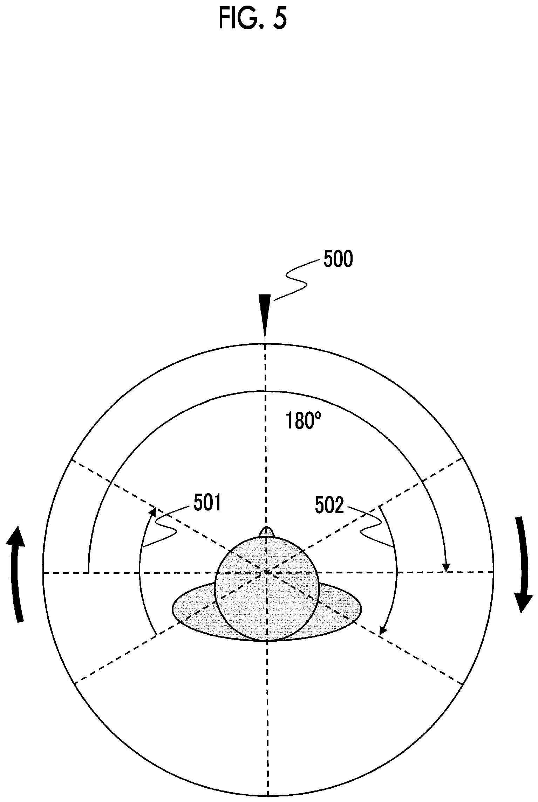

Fig. 5 shows the relationship between the target reconstructed image position, and the first image and the second image. In Fig. 5, a position 500 indicates a target image reconstruction center position set in the image reconstruction condition setting step S42, and in this example, a position (position at a rotation angle of 0° of the scanner) where the X-ray tube and the subject 3 are directly opposite is the target image reconstruction center position. In the reconstruction of the tomographic image, the transmitted X-ray data in a predetermined angular range centered on the position 500 (0°), for example, a range of 180° or more, is used, but the image pair is generated by the transmitted X-ray data having ranges 501 and 502 respectively centered on positions of -90° and +90° as image reconstruction ranges. That is, the sizes of the image reconstruction ranges 501 and 502 are equal, each being a range of an angle of less than 180°, and their image reconstruction center positions are 180° apart from each other. As this angular range, a predetermined value as a default value may be set, or user settings/changes may be accepted in the image reconstruction condition setting step S42.

Tube Current Ratio Calculation: S44

Next, the processing condition adjustment unit 335 (tube current ratio calculation unit) acquires information on the tube current in a case where each piece of transmitted X-ray data of the first image and the second image has been acquired, and calculates the ratio between these tube currents (tube current ratio). Specifically, the information on the tube current is acquired from the transmitted X-ray data collected in the imaging step S41. Although imaging may be performed with a constant tube current, for example, in imaging of the heart, in order to reduce the radiation exposure dose, the tube current may be changed based on the electrocardiogram waveform data during imaging, between a stationary phase in which a reconstructed image is created and other phases. The present embodiment is applied to imaging involving such changes in the tube current.

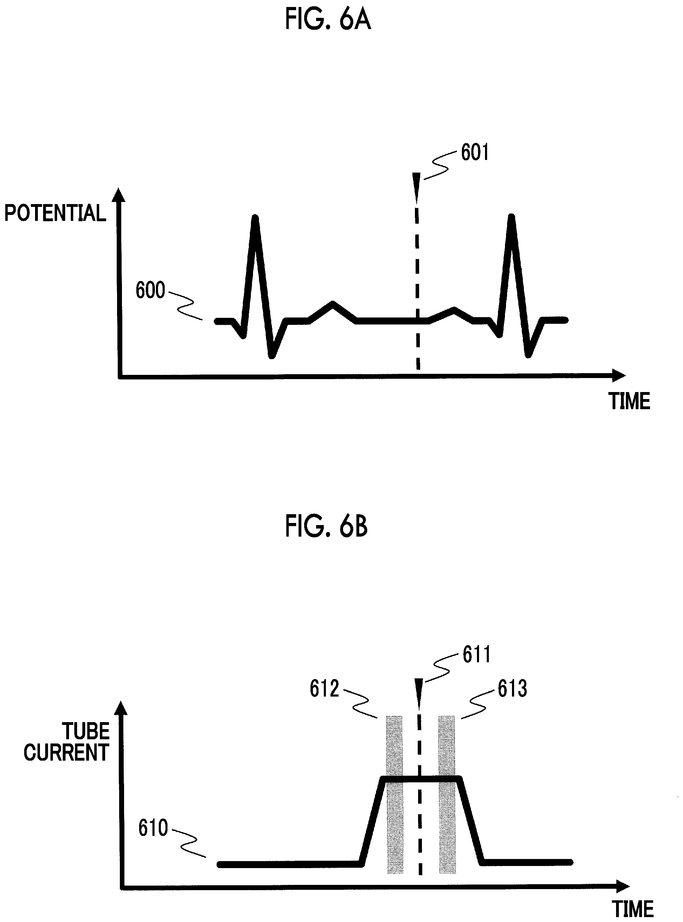

Figs. 6A to 6C show examples of the relationship between electrocardiogram waveform data (600) and the magnitude (610 and 620) of the tube current value in a case where the tube current is changed. In the electrocardiogram waveform 600 shown in Fig. 6A, in a case where an imaging target cardiac phase 601 is set between an R-wave and an R-wave adjacent to each other, the tube current is set such that the center of the waveform is aligned with the imaging target cardiac phase 601 as shown in Fig. 6B.

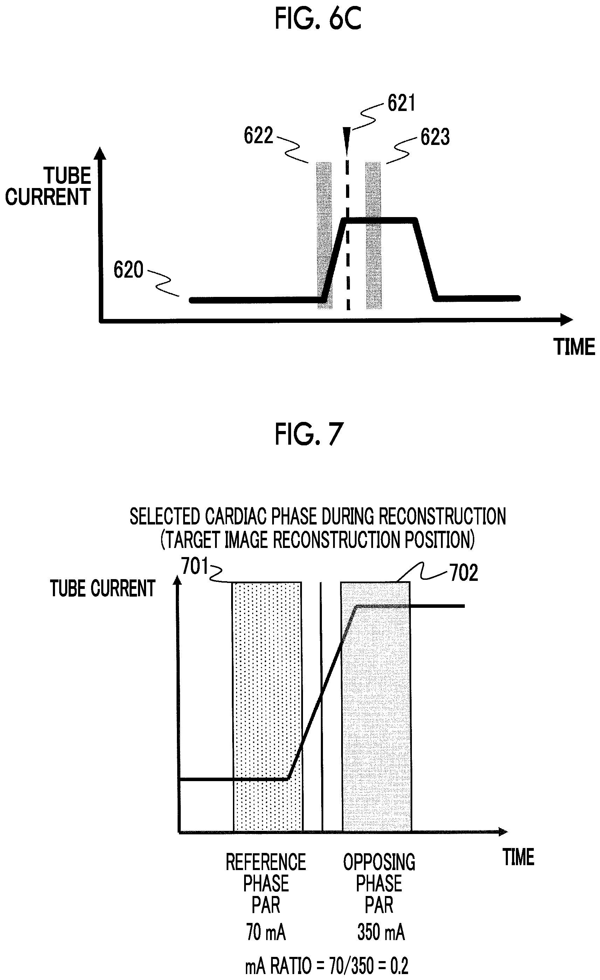

Here, in a case where a selected cardiac phase during reconstruction (that is, the target image reconstruction position) 611 set in the image reconstruction condition setting step S42 matches the imaging target cardiac phase 601, the tube current values in an image reconstruction range 612 of the first image and an image reconstruction range 613 of the second image, which are equidistant (at an equal time interval) from the position 611, are equal, but as shown in Fig. 6C, in a case where the selected cardiac phase during reconstruction (target image reconstruction position) 621 is shifted by some extent from the imaging target cardiac phase 601, the tube current value in an image reconstruction range 622 of the first image and the tube current value in an image reconstruction range 623 of the second image do not match.

In a case where the tube currents of the two images constituting the image pair are significantly different from each other as described above, the acquisition of the motion information of the subject using the images is inaccurate, which causes over-correction even in a case where the motion-corrected image reconstruction is performed. Therefore, in the present embodiment, the conditions of the filtering processing and the registration processing for acquiring the motion information, which will be described below, are adjusted by using the calculated tube current ratio, thereby preventing the motion correction from being inaccurate.

The tube current ratio calculated by the tube current ratio calculation unit is a value obtained by dividing an average tube current value of the first image by an average tube current value of the second image, or a reciprocal of this value. For example, in the example shown in Fig. 7, in a case where the average tube current of an image (first image) 701 at a reference phase for motion correction is 70 mA and the average tube current of an image (second image) 702 at a phase corresponding to the reference phase is 350 mA, the tube current ratio (mA ratio) is 0.2.

The processing condition adjustment unit 335 classifies the image pair based on the calculated tube current ratio and adjusts the parameters of noise reduction processing S45 and registration processing S46 in accordance with the classification. Details of the adjustment will be described below.

Noise Reduction Processing: S45

The filtering unit 331 performs filtering on the first image and the second image generated in the image pair generation step S43. For filtering, a smoothing filter such as a low-pass filter, a high-pass filter, a Gaussian filter, or a bilateral filter can be used, and the filtering unit 331 uses one or more of these as the smoothing filter to perform noise reduction. In this case, the characteristics (parameters) of the filter to be used are adjusted based on the tube current ratio calculated in step S44.

Smoothing parameters to be adjusted vary depending on the filter to be used. As an example, adjustable parameters in a case of using a bilateral filter will be described.

The bilateral filter is represented by Equation (1), in which three parameters, w, $\sigma_1$, and $\sigma_2$, are parameters for determining the strength of smoothing. The strength of smoothing is determined by adjusting one or more of these parameters. In a case of increasing smoothing, the values of w, $\sigma_1$, and $\sigma_2$ are each adjusted in a direction of increasing the value, and in a case of decreasing smoothing, the values of w, $\sigma_1$, and $\sigma_2$ are each adjusted in a direction of decreasing the value.

$$g(i,j) = \frac{\sum_{n=-w}^{w} \sum_{m=-w}^{w} f(i+m,j+n) \exp\left(-\frac{m^2+n^2}{2\sigma_1^2}\right) \exp\left(-\frac{(f(i,j)-f(i+m,j+n))^2}{2\sigma_2^2}\right)}{\sum_{n=-w}^{w} \sum_{m=-w}^{w} \exp\left(-\frac{m^2+n^2}{2\sigma_1^2}\right) \exp\left(-\frac{(f(i,j)-f(i+m,j+n))^2}{2\sigma_2^2}\right)}$$

$$(1)$$

In Equation (1), f(i,j) represents an array of input image data, g(i,j) represents an array of output image data, w represents kernel size, $\sigma_1$ represents a weight in which a distance from a target voxel is taken into consideration, and $\sigma_2$ represents a weight in which a difference in the pixel value from the target voxel is taken into consideration.

Here, since various values can be taken as the tube current ratio depending on the timing at which the first image and the second image are acquired, it is not easy to individually adjust these parameters for all the tube current ratios. In the present embodiment, the tube current ratio is divided into a plurality of ranges in advance by threshold values, and a value of the parameter, a function for determining the value, or the like is set in advance for each range. The parameters to be set in advance can be set by, for example, using statistical data such as past clinical data to obtain a value at which smoothing without over-correction can be achieved, and setting the obtained value as the value of the parameter. The value of the parameter for each set range is stored in the storage device 213 of the calculation unit 30, for example, as a table.

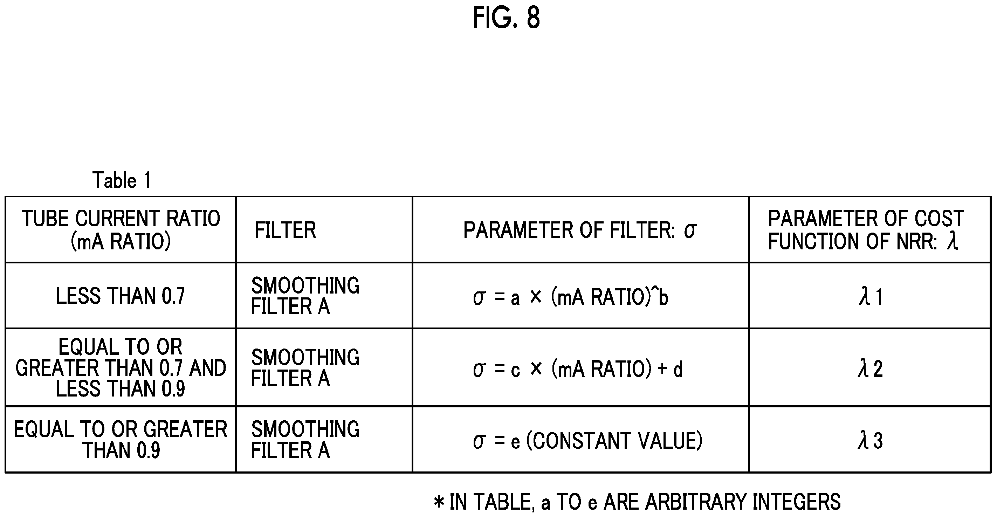

Fig. 8 shows an example (Table 1) of a table in which, as an example, two values, 0.7 and 0.9, are used as threshold values of the tube current ratio (the ratio of the tube current of the image at the opposing phase with respect to the tube current of the image at the reference phase), the range of the tube current ratio is divided into three ranges, "mA ratio < 0.7", "0.7 ≤ mA ratio < 0.9", and "mA ratio ≥ 0.9", and parameters corresponding to each range are set. In this example, a parameter set including the parameter of the registration processing, which will be described below, is set in each range of the tube current ratio.

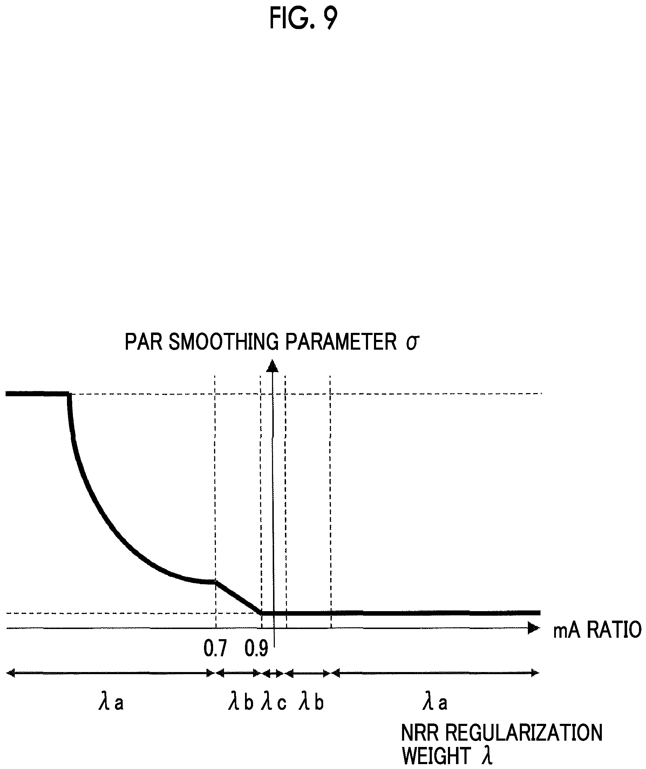

In addition, regarding the filtering, in an example in which $\sigma$ ($\sigma_1$, $\sigma_2$) among three parameters (w, $\sigma_1$, $\sigma_2$) shown in Equation (1) is set, the parameter $\sigma$ is set to be a non-linear function of the tube current ratio in a case of "mA ratio < 0.7", a linear function of the tube current ratio in a case of "0.7 ≤ mA ratio < 0.9", and a fixed value in a case of "mA ratio ≥ 0.9". Fig. 9 is a graph showing the characteristics of the filter adjusted in accordance with this setting. The parameter set is not limited to this example, and other parameters w may also be set for each range. Further, in Table 1, among the conditions of the filtering, the type of filter is fixed, and only the parameters are varied for each classification, but the type of filter may be varied for each classification.

As shown in Fig. 9, by making the filter characteristics asymmetric with respect to the mA ratio = 1.0, different filtering is applied to each of the first image and the second image in accordance with the tube current thereof.

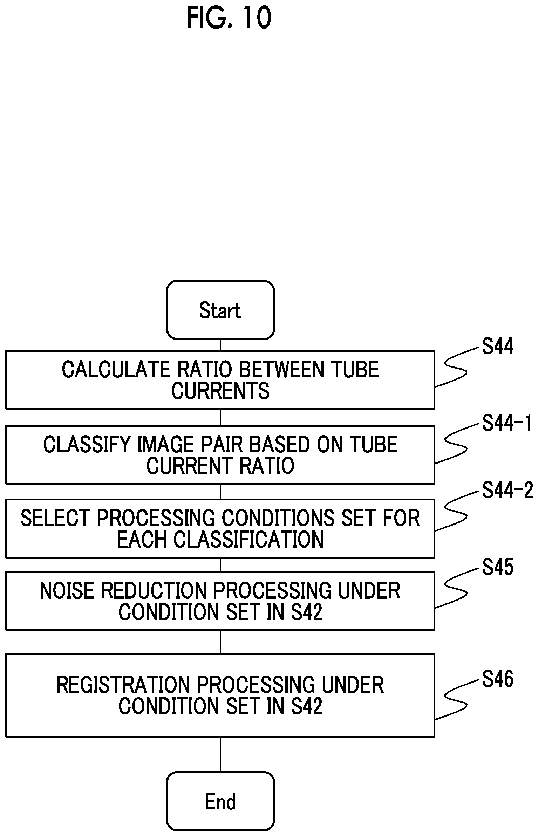

As shown in Fig. 10, the following processes are added to the adjustment of the parameters in a case where the parameters are set in advance for each range of the tube current ratio: classifying the image pair based on which range the tube current ratio calculated for a predetermined image pair falls into (S44-1); and selecting predetermined parameters for each classification (for each range of the tube current ratio) (S44-2), after the tube current ratio is calculated (S44).

In this way, by classifying the image pair in accordance with the tube current ratio and selecting the predetermined parameter based on the classification, the parameter adjustment can be easily achieved.

Registration Processing: S46

After the noise reduction processing by the filtering unit mentioned above, the registration processing using the image pair is performed, and the motion information is acquired. For example, in the registration processing, non-rigid registration of the second image is performed with the first image as a reference, and a deformation function for minimizing the error between the first image and the second image is obtained. In the present embodiment, a parameter (weight $\lambda$ of the regularization term) for adjusting the degree of consideration of the regularization term in the cost function in the calculation of the non-rigid registration is adjusted in accordance with the tube current ratio.

In the non-rigid registration, a free-form deformation (FFD) model based on a B-spline function is used as a motion model. In a case where a 3D FFD model is used, the deformation function is represented by Equation (2).

$$T_j(x; \Theta) = x + \sum_j B\left(\frac{x}{d} - j\right) \Theta_j$$

$$(2)$$

In Equation (2), T is an FFD model based on 3D B-splines, x is a voxel, j is a control point of interest, B is a third-order tensor product consisting of cubic B-splines, d is an interval between control points in a spatial domain, $\Theta_j$ is a displacement vector (3D) of the control point of interest j, and $\Theta$ is a set of displacement vectors (3D) of the control points representing a motion relationship between a reference time point and each time point.

The parameter at the control point of the deformation function is obtained by minimizing the dissimilarity (Equation (3)) based on the squared error (SSD) between the first image at the reference phase and the second image at the phase opposing the reference phase.

$$D(\Theta) = \sum_x \left| P_{target}(x) - P_{source}\left(T_j(x;\Theta)\right) \right|^2 \qquad (3)$$

In Equation (3), D(O) represents the dissimilarity based on the SSD, $P_{target}(x)$ represents the image (first image) at the reference phase, and $P_{source}(x)$ represents the image (second image) at the phase 180° apart from the reference phase.

Here, the convergence calculation for the minimization is a poorly posed problem having a large number of transformation parameters, and a regularization term, as represented by Equation (4), is introduced in order to efficiently and robustly solve the problem.

$$R(\Theta) = \sum_j \sum_{j' \in K_j} \left\| \theta_j - \theta_{j'} \right\|_2^2 \qquad (4)$$

In Equation (4), R($\Theta$) is a regularization term that penalizes the difference in parameters between spatially adjacent control points, and $K_j$ represents a set of indices for control points spatially adjacent to the j-th control point.

Equation (4) is a regularization term that penalizes the difference in parameters between the spatially adjacent control points. That is, the term is used to suppress excessive deformation in the image of interest. The cost function of the non-rigid registration is represented by Equation (5) using the dissimilarity D(O) represented by Equation (3) and the regularization term R($\Theta$) represented by Equation (4).

$$C(\Theta) = D(\Theta) + \lambda R(\Theta)$$
$$= \sum_x \left| P_{target}(x) - P_{source}\left(T_j(x;\Theta)\right) \right|^2 + \lambda \sum_j \sum_{j' \in K_j} \left\| \theta_j - \theta_{j'} \right\|_2^2 \qquad (5)$$

In Equation (5), $\lambda$ is a weight parameter of the regularization term R(O), and in the present embodiment, the weight $\lambda$ is adjusted in accordance with the tube current ratio.

In adjusting the weight, similar to adjusting the parameter of filtering corresponding to the tube current ratio, the value of the weight is determined in advance for each classification in accordance with the range of the tube current ratio, and the calculation of the non-rigid registration mentioned above is executed with the weight $\lambda$ predetermined in accordance with the range (classification) to which the tube current ratio of the image pair belongs. As shown in Table 1 of Fig. 8 described above, the weight $\lambda$ predetermined in accordance with the classification is stored in the table, and the registration unit reads out the weight corresponding to the tube current ratio of the image pair from the table in a case of executing the non-rigid registration.

Through this registration processing, a motion vector between the images is calculated.

Motion-Corrected Image Reconstruction: S47

In the motion-corrected image reconstruction, image reconstruction is performed by using the transmitted X-ray data obtained in the imaging step S41 and the motion information, that is, the motion vector, acquired in the above step.

In the motion-corrected image reconstruction, the magnitude or the direction of the motion of the subject at the time of acquisition of each piece of transmitted X-ray data used for the image reconstruction is estimated from the motion vectors calculated from the first image and the second image created at positions 180° apart from each other with the target reconstruction position as the center, and back projection is performed while correcting the image at the target reconstruction position based on this information, thereby reconstructing a tomographic image.

By performing this processing for each of a plurality of cross-sections, motion-corrected 3D tomographic image data is obtained. The obtained tomographic image is stored in the storage device 213 as necessary and is displayed on the display device 211.

As described above, according to the present embodiment, by adjusting the conditions of the filtering and the registration performed in a case of motion detection by using the tube current ratio in a case where each image of the image pair for detecting motion has been acquired, over-correction caused by the difference in the tube currents between the image pair can be suppressed, and the occurrence of streak artifacts and the distortion of the blood vessel shape can be reduced. In particular, by adjusting the parameters of the cost function not only for filtering but also for registration, it is possible to prevent a decrease in the accuracy of registration even in a case where the tube current ratio deviates significantly from 1 and to acquire highly accurate motion information.

Further, according to the present embodiment, by dividing the ratio between the tube current of the first image and the tube current of the second image into a plurality of ranges and setting the parameters to be adjusted for each range in advance, the parameter adjustment can be easily achieved.

In the above-mentioned embodiment, the conditions of both the filtering and the registration are adjusted in accordance with the tube current ratio, but the present invention also includes adjusting only the parameter of the registration processing.

Embodiment 2

In Embodiment 1, the registration processing is performed after the filtering processing on the image pair, but the present embodiment is characterized in that the presence or absence of motion is determined by using the image pair before detecting the motion in the registration processing, and the pixel value normalization is performed in accordance with the determination result.

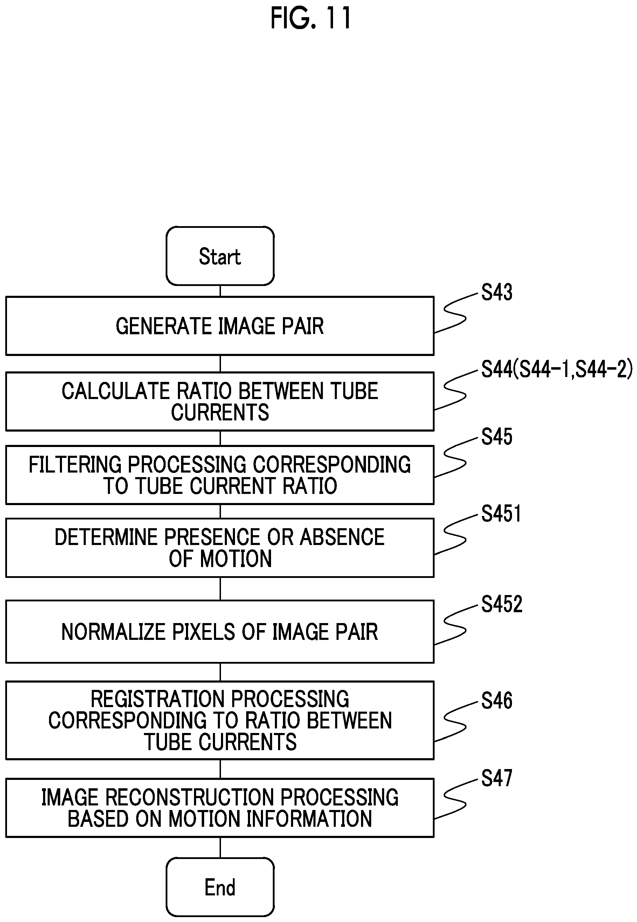

Hereinafter, processing of the present embodiment will be described with reference to Fig. 11. In Fig. 11, the same processing as the processing shown in Fig. 4 is denoted by the same reference numerals, and duplicated descriptions will not be repeated.

After performing filtering on the image pair under the conditions corresponding to the tube current ratio of the image pair (S43 to S45), the presence or absence of motion is determined by using the difference between the image pair (the first image and the second image). Hereinafter, the details will be described.

Determination of Presence or Absence of Motion: S451

In this step, first, an image difference is acquired for the first image and the second image acquired in the image pair generation step S43, or for the first image and the second image after the filtering in the noise reduction processing S45. The first image and the second image are generated for each of the plurality of cross-sections in a case of a three-dimensional image. That is, a plurality of image pairs are obtained, and a plurality of difference images are also obtained. A standard deviation of pixel values is calculated for each image of all the acquired difference images, and a median value of the standard deviations of all the difference images is set as a representative value of the standard deviations of the image pairs. That is, the representative value of the standard deviations serves as an indicator of the motion of the acquired three-dimensional image as a whole.

Next, the presence or absence of motion of the subject is determined based on the calculated value of the standard deviation. Specifically, it is determined that there is motion in the subject in a case where the value of the standard deviation is equal to or greater than a threshold value, and it is determined that there is no motion in the subject in a case where the value of the standard deviation is less than the threshold value.

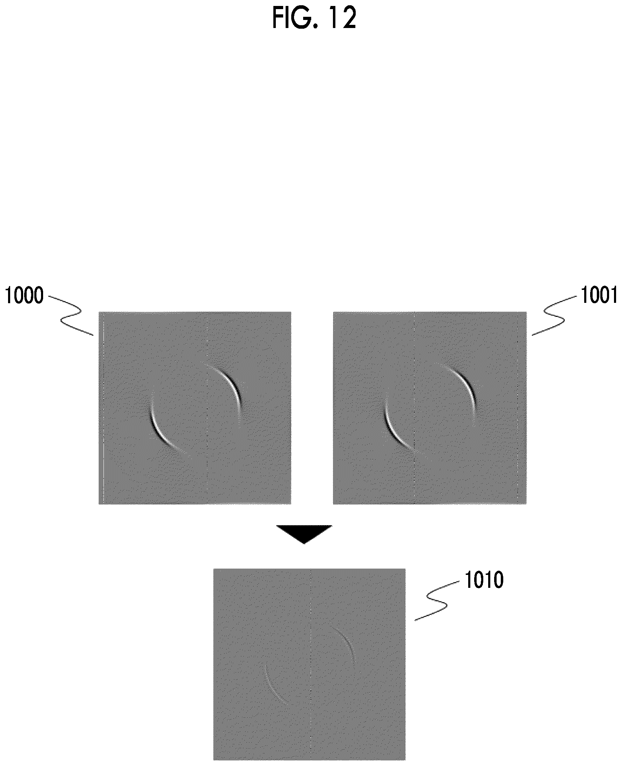

Fig. 12 shows a determination example of the presence or absence of motion with respect to a motionless subject. In Fig. 12, a difference image 1010 is a difference image between a first image 1000 and a second image 1001 after filtering. As shown in Fig. 12, the pixel values of the difference image 1010 themselves are low, and the value of the standard deviation to be calculated from the pixel values is below the threshold value. Therefore, it is determined that there is no motion in the subject.

Normalization: S452

Pixel value normalization processing is performed on the first image and the second image after filtering based on the result of the determination (S451) of the presence or absence of motion. The normalization is performed on all the pixels of the first image and the second image by using, for example, a Min-Max method, but different processing is performed in accordance with the determination result in the determination step S451.

The image in which it is determined that there is motion in the subject is normalized to a range of a minimum value of 0 and a maximum value of 1 by applying Equation (6) to the input image.

The image in which it is determined that there is no motion in the subject is normalized to a range of a minimum value of 0 and a maximum value of M ($0 < M \leq 1$) by applying a value, which is obtained by increasing the denominator ($f_{max} - f_{min}$) of Equation (6) to a predetermined constant value T ($T \geq f_{max} - f_{min}$), to the input image.

$$g(i,j) = \frac{f(i,j) - f_{min}}{f_{max} - f_{min}} \qquad (6)$$

$f(i,j)$: array of input image data, $g(i,j)$: array of output image data,

$f_{min}$: minimum value of pixel values of input image. $f_{max}$: maximum value of pixel values of input image

In this way, the presence or absence of motion is determined, and the normalization method is varied based on the determination result, that is, the normalized value is adjusted to be smaller in a case where there is no motion, thereby adjusting the strength of the motion correction. Consequently, it is possible to suppress excessive motion correction performed even in a case where there is no motion.

After that, the registration processing is performed with the parameter corresponding to the tube current ratio by using the normalized image pair (S46), and the motion-corrected image reconstruction is performed (S47) in the same manner as in Embodiment 1.

According to the present embodiment, by determining the presence or absence of motion of the subject and changing conditions in a case of normalizing two images depending on the degree (presence or absence) of motion, it is possible to solve problems such as excessive correction being performed despite minimal motion and unnatural distortion caused by image noise.

The embodiments of the X-ray CT apparatus and the motion-corrected image reconstruction according to the embodiment of the present invention have been described above, and the present invention is characterized in that the conditions of the noise reduction processing and the registration are adjusted by using the tube current ratio of the image pair for detecting the motion information, and suitably, the tube current ratio is divided into a plurality of ranges by threshold values, and the predetermined condition is selected and applied for each range. The present invention is not limited to these embodiments, and various changes can be made.

Explanation of References

1: X-ray CT apparatus
3: subj ect
10: imaging unit
20: operation unit
30: calculation unit
100: gantry
101: patient table device
102: X-ray generation device
103: X-ray detection device
104: collimator device
105: high-voltage generation device
107: drive device
106: data collection device
200: central control unit
210: input/output device
211: display device
212: input device
213: storage device
310: image reconstruction unit
320: image pair generation unit
330: motion information acquisition unit
335: processing condition adjustment unit
340: motion-corrected image reconstruction unit

**Claims**

1. An X-ray CT apparatus comprising:

    an imaging unit (10) that includes an X-ray source (102) and an X-ray detector (103) which rotate around a subject (3) and that acquires transmitted X-ray data of the subject in a predetermined angular range; and
    one or more processors (200) that generate a reconstructed image by using the transmitted X-ray data acquired by the imaging unit,
    wherein the one or more processors are configured to
    generate a first image and a second image at directly opposite positions by using a part of the transmitted X-ray data,
    perform noise reduction processing on the first image and the second image and registration processing between the first image and the second image after noise reduction and calculate motion information of the subject during scanning,
    classify an image pair consisting of the first image and the second image, in accordance with a ratio between tube currents in a case where the transmitted X-ray data used to generate the first image and the second image has been acquired, during the calculation of the motion information, and adjust conditions of the noise reduction processing and the registration processing for each classification, and
    the one or more processors generate the reconstructed image by correcting motion of the subject during scanning using the calculated motion information.

2. The X-ray CT apparatus according to claim 1,

    wherein the imaging unit (10) incorporates information from an electrocardiograph attached to the subject and performs electrocardiogram-synchronized imaging with a desired cardiac phase as a reconstruction cardiac phase, and
    transmitted X-ray data of the first image and transmitted X-ray data of the second image are at directly opposite positions with the reconstruction cardiac phase as a center.

3. The X-ray CT apparatus according to claim 1,

    wherein the imaging unit (10) performs asynchronous imaging under automatic exposure control, and
    transmitted X-ray data of the first image and transmitted X-ray data of the second image are at directly opposite positions with respect to a reconstruction center position designated by a user.

4. The X-ray CT apparatus according to any preceding claim,
    wherein the one or more processors (200) set two or more threshold values for the ratio between the tube currents and classify the image pair into three or more classifications.

5. The X-ray CT apparatus according to claim 4,
    wherein the transmitted X-ray data acquired by the imaging unit is three-dimensional data that includes a slice direction intersecting a rotation direction, and the one or more processors generate a plurality of the image pairs along the slice direction.

6. The X-ray CT apparatus according to any preceding claim,

    wherein the one or more processors include a smoothing filter, and
    adjust at least one of a type or a parameter of the smoothing filter for each classification as the condition of the noise reduction processing.

7. The X-ray CT apparatus according to claim 6,
    wherein the one or more processors vary the condition of the noise reduction processing for the first image and the condition of the noise reduction processing for the second image.

8. The X-ray CT apparatus according to any preceding claim,

    wherein the one or more processors include a non-rigid registration algorithm, and
    adjust a parameter of the non-rigid registration algorithm for each classification as the condition of the registration processing.

9. The X-ray CT apparatus according to any preceding claim,

wherein the one or more processors include a noise reduction filter and a non-rigid registration algorithm, and adjust at least one of a type or a parameter of the noise reduction filter and a parameter of the non-rigid registration algorithm as the conditions of the noise reduction processing and the registration processing.

10. An X-ray CT apparatus comprising:

an imaging unit (10) that includes an X-ray source (102) and an X-ray detector (103) which rotate around a subject (3) and that acquires transmitted X-ray data of the subject in a predetermined angular range; and one or more processors (200) that generate a reconstructed image by using the transmitted X-ray data acquired by the imaging unit,
wherein the one or more processors are configured to

generate a first image and a second image at directly opposite positions by using a part of the transmitted X-ray data, and
perform registration processing on the first image and the second image based on a non-rigid registration algorithm and calculate motion information of the subject during scanning,
adjust a parameter of the non-rigid registration algorithm, in accordance with a ratio between tube currents in a case where the transmitted X-ray data used to generate the first image and the second image has been acquired, during the calculation of the motion information, and

the one or more processors generate the reconstructed image by correcting motion of the subject during scanning using the calculated motion information.

11. The X-ray CT apparatus according to claim 10,

wherein the one or more processors include a noise reduction filter used in noise reduction processing on the first image and the second image prior to the registration processing, and
adjust at least one of a type or a parameter of the noise reduction filter, in accordance with the ratio between the tube currents in a case where the transmitted X-ray data used to generate the first image and the second image has been acquired.

12. An image processing device that accepts transmitted X-ray data collected by an X-ray CT apparatus as claimed in any of claims 1 to 9 and that performs motion-corrected image reconstruction, the image processing device comprising: wherein the image processing device include the one or more processors of the apparatus according to any of claims 1 to 9 as a processor that performs motion-corrected image reconstruction.

13. An image processing device that accepts transmitted X-ray data collected by an X-ray CT apparatus of claim 10 or 11 and that performs motion-corrected image reconstruction, the image processing device comprising: wherein the image processing device include the one or more processors of the apparatus according to claim 10 or 11 as a processor that performs motion-corrected image reconstruction.

14. A motion-corrected image reconstruction method of performing image reconstruction of a CT image by correcting motion of a subject during scanning by using transmitted X-ray data, the method comprising:

generating a first image and a second image at directly opposite positions by using a part of the transmitted X-ray data;
acquiring motion information of the subject by performing noise reduction processing and registration processing after noise reduction, on each of the first image and the second image;
adjusting, in the acquisition, at least one of a type or a parameter of a noise reduction filter used in the noise reduction processing and a parameter of a non-rigid registration algorithm used in the registration processing, based on a ratio between tube currents in a case where the first image and the second image have been acquired; and
performing image reconstruction by using the motion information and the transmitted X-ray data.

# FIG. 1

EP 4 537 759 A1

## FIG. 2

HIGH-VOLTAGE GENERATION DEVICE

DATA COLLECTION DEVICE

DISPLAY DEVICE

INPUT DEVICE

STORAGE DEVICE

CPU

IMAGE RECONSTRUCTION UNIT (MOTION-CORRECTED IMAGE RECONSTRUCTION)

EP 4 537 759 A1

# FIG. 3

IMAGE RECONSTRUCTION UNIT — 310

IMAGE PAIR GENERATION UNIT — 320

MOTION INFORMATION ACQUISITION UNIT — 330

FILTERING UNIT — 331

REGISTRATION UNIT — 333

PROCESSING CONDITION ADJUSTMENT UNIT — 335

335

TUBE CURRENT RATIO CALCULATION UNIT

PARAMETER SET TABLE

MOTION-CORRECTED IMAGE RECONSTRUCTION UNIT — 340

EP 4 537 759 A1

# FIG. 4

Start

ACQUIRE TRANSMITTED X-RAY DATA — S41

SET IMAGE RECONSTRUCTION CONDITIONS — S42

GENERATE IMAGE PAIR — S43

CALCULATE RATIO BETWEEN TUBE CURRENTS — S44

NOISE REDUCTION PROCESSING CORRESPONDING TO RATIO BETWEEN TUBE CURRENTS — S45

REGISTRATION PROCESSING CORRESPONDING TO RATIO BETWEEN TUBE CURRENTS — S46

IMAGE RECONSTRUCTION PROCESSING BASED ON MOTION INFORMATION — S47

End

# FIG. 5

## FIG. 6A

## FIG. 6B

# FIG. 6C

# FIG. 7

SELECTED CARDIAC PHASE DURING RECONSTRUCTION
(TARGET IMAGE RECONSTRUCTION POSITION)

REFERENCE
PHASE
PAR

OPPOSING
PHASE
PAR

70 mA

350 mA

mA RATIO = 70/350 = 0.2

# FIG. 8

Table 1

| TUBE CURRENT RATIO (mA RATIO) | FILTER | PARAMETER OF FILTER: $\sigma$ | PARAMETER OF COST FUNCTION OF NRR: $\lambda$ |
|---|---|---|---|
| LESS THAN 0.7 | SMOOTHING FILTER A | $\sigma = a \times (\text{mA RATIO})\hat{\ }b$ | $\lambda 1$ |
| EQUAL TO OR GREATER THAN 0.7 AND LESS THAN 0.9 | SMOOTHING FILTER A | $\sigma = c \times (\text{mA RATIO}) + d$ | $\lambda 2$ |
| EQUAL TO OR GREATER THAN 0.9 | SMOOTHING FILTER A | $\sigma = e$ (CONSTANT VALUE) | $\lambda 3$ |

\* IN TABLE, a TO e ARE ARBITRARY INTEGERS

EP 4 537 759 A1

# FIG. 9

PAR SMOOTHING PARAMETER $\sigma$

0.7  0.9

mA RATIO

$\lambda$ a    $\lambda$ b $\lambda$ c $\lambda$ b    $\lambda$ a

NRR REGULARIZATION
WEIGHT $\lambda$

# FIG. 10

```
                    ┌──────────┐
                    │  Start   │
                    └──────────┘
                          │
    ┌─────────────────────────────────────┐      S44
    │  CALCULATE RATIO BETWEEN TUBE        │
    │           CURRENTS                   │
    └─────────────────────────────────────┘
                          │
    ┌─────────────────────────────────────┐      S44-1
    │  CLASSIFY IMAGE PAIR BASED ON TUBE   │
    │          CURRENT RATIO               │
    └─────────────────────────────────────┘
                          │
    ┌─────────────────────────────────────┐      S44-2
    │  SELECT PROCESSING CONDITIONS SET FOR│
    │         EACH CLASSIFICATION          │
    └─────────────────────────────────────┘
                          │
    ┌─────────────────────────────────────┐      S45
    │  NOISE REDUCTION PROCESSING UNDER    │
    │       CONDITION SET IN S42           │
    └─────────────────────────────────────┘
                          │
    ┌─────────────────────────────────────┐      S46
    │  REGISTRATION PROCESSING UNDER       │
    │       CONDITION SET IN S42           │
    └─────────────────────────────────────┘
                          │
                    ┌──────────┐
                    │   End    │
                    └──────────┘
```

# FIG. 11

Start

GENERATE IMAGE PAIR — S43

CALCULATE RATIO BETWEEN TUBE CURRENTS — S44(S44-1,S44-2)

FILTERING PROCESSING CORRESPONDING TO TUBE CURRENT RATIO — S45

DETERMINE PRESENCE OR ABSENCE OF MOTION — S451

NORMALIZE PIXELS OF IMAGE PAIR — S452

REGISTRATION PROCESSING CORRESPONDING TO RATIO BETWEEN TUBE CURRENTS — S46

IMAGE RECONSTRUCTION PROCESSING BASED ON MOTION INFORMATION — S47

End

# FIG. 12

1000

1001

1010

**EP 4 537 759 A1**

# EUROPEAN SEARCH REPORT

**Application Number**

EP 24 20 6505

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | US 10 165 989 B2 (SAMSUNG ELECTRONICS CO LTD [KR]) 1 January 2019 (2019-01-01) * column 6, line 27 - column 19, line 35 * * figures 1,2,4A,5,6,10,11 * ----- | 1-14 | INV. A61B6/03 A61B6/50 A61B6/00 G06T11/00 G06T5/70 |
| A | US 2016/317102 A1 (ISHII SUNAO [JP] ET AL) 3 November 2016 (2016-11-03) * paragraph [0008]; figures 1,3,10,12,13,15 * * paragraph [0040] * * paragraph [0043] * * paragraph [0057] - paragraph [0060] * * paragraph [0074] - paragraph [0077] * ----- | 1-14 | |
| A | US 2005/078791 A1 (LI JIANYING [US]) 14 April 2005 (2005-04-14) * paragraph [0001] - paragraph [0044] * * figures 1-5 * ----- | 1-14 | |
| X,P | US 2023/329662 A1 (TETSUMURA YUSUKE [JP] ET AL) 19 October 2023 (2023-10-19) * the whole document * ----- | 1-7,12 | |
| | | | **TECHNICAL FIELDS SEARCHED (IPC)** A61B G06T |

The present search report has been drawn up for all claims

| Place of search | Date of completion of the search | Examiner |
|---|---|---|
| Munich | 3 February 2025 | De la Hera, Germán |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 6505

This annex lists the patent family members relating to the patent documents cited in the above-mentioned European search report.
The members are as contained in the European Patent Office EDP file on
The European Patent Office is in no way liable for these particulars which are merely given for the purpose of information.

03-02-2025

| Patent document cited in search report | | | Publication date | Patent family member(s) | | | Publication date |
|---|---|---|---|---|---|---|---|
| US 10165989 | B2 | | 01-01-2019 | KR | 20170030308 | A | 17-03-2017 |
| | | | | US | 2017069061 | A1 | 09-03-2017 |
| US 2016317102 | A1 | | 03-11-2016 | CN | 105873519 | A | 17-08-2016 |
| | | | | JP | 6509131 | B2 | 08-05-2019 |
| | | | | JP | WO2015108097 | A1 | 23-03-2017 |
| | | | | US | 2016317102 | A1 | 03-11-2016 |
| | | | | WO | 2015108097 | A1 | 23-07-2015 |
| US 2005078791 | A1 | | 14-04-2005 | NONE | | | |
| US 2023329662 | A1 | | 19-10-2023 | CN | 116898465 | A | 20-10-2023 |
| | | | | JP | 2023156873 | A | 25-10-2023 |
| | | | | US | 2023329662 | A1 | 19-10-2023 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023176865 A

- US 10165989 B2